# EUROPEAN PATENT SPECIFICATION

(11) **EP 3 471 706 B1**
(45) Date of publication and mention of the grant of the patent: **20.12.2023**
(21) Application number: 17732089.2
(22) Date of filing: 20.06.2017
(51) Int. Cl.: A61K 9/16, A61K 39/02, A61K 39/09, A61K 39/108, A61K 39/00, B01J 2/06, A61K 9/50

(54) **ORAL PHARMACEUTICAL COMPOSITION COMPRISING A PHARMACEUTICALLY ACTIVE AGENT, AT LEAST ONE CATIONIC BIOADHESIVE POLYMER AND AT LEAST TWO ANIONIC POLYMERS**
ORALE PHARMAZEUTISCHE ZUSAMMENSETZUNG MIT EINEM PHARMAZEUTISCH AKTIVEN WIRKSTOFF, MINDESTENS EINEM KATIONISCHEN BIOADHÄSIVEN POLYMER UND MINDESTENS ZWEI ANIONISCHEN POLYMEREN
COMPOSITION PHARMACEUTIQUE ORALE COMPRENANT UN AGENT PHARMACEUTIQUEMENT ACTIF, AU MOINS UN POLYMÈRE BIOADHÉSIF CATIONIQUE ET AU MOINS DEUX POLYMÈRES ANIONIQUES

(30) Priority: 20.06.2016 EP 16175308
(43) Date of publication of application: 24.04.2019
(73) Proprietor: VIRBAC, 06516 Carros (FR)
(72) Inventor: DELHOM, Nathalie, 06140 Vence (FR)
(74) Representative: Gevers & Orès
(86) International application number: PCT/EP2017/065137
(87) International publication number: WO 2017/220611

(56) References cited:
- WO-A1-99/18934
- WO-A1-2005/013941
- WO-A1-2011/026896
- WO-A1-2015/084594
- US-A1- 2009 238 845
- YI-CHENG HUANG ET AL: "Mobilization of mesenchymal stem cells by stromal cell-derived factor-1 released from chitosan/tripolyphosphate/fucoidan nanoparticles", ACTA BIOMATERIALIA, vol. 8, no. 3, 13 December 2011 (2011-12-13), pages 1048-1056, XP055325562, AMSTERDAM, NL ISSN: 1742-7061, DOI: 10.1016/j.actbio.2011.12.009
- MARTINS ET AL: "Insulin-loaded alginate microspheres for oral delivery - Effect of polysaccharide reinforcement on physicochemical properties and release profile", CARBOHYDRATE POLYMERS, APPLIED SCIENCE PUBLISHERS, LTD. BARKING, GB, vol. 69, no. 4, 23 May 2007 (2007-05-23), pages 725-731, XP022093496, ISSN: 0144-8617, DOI: 10.1016/J.CARBPOL.2007.02.012

## Description

The present invention refers to the field of oral delivery vehicle allowing the release of pharmaceutically active agent at intestinal level.

The present invention relates to a solid oral composition comprising a pharmaceutically active agent, such as, an immunogenic agent (e.g., a vaccine), at least one cationic bioadhesive polymer which is preferably chitosan and at least two anionic compounds that allows the oral administration and delivery of the pharmaceutically active agent essentially unaltered to the intestinal mucosa.

As some biologically active substances are easily degradable in an acid environment and/or are suitable for an immune response production, intestinal absorption can be recommended. It is indeed known that the intestinal mucosa has a large number of lymphoid cells able to produce an effective immune response to external infective stimuli (Van der Lubben I. M. et al., 2001 Adv. Drug. Del. Rev., 52: 139-144; Schep L. J. et al., 1999 J. Contr. Release, 59 : 1-14).

Consequently, the development of efficient oral carrier systems for these kinds of biologically active substances needs particular attention to release at intestinal level, said intestinal release may be associated with the ability to adhere to the mucosa of the compositions employed to carry said substances.

The mechanism of adhesion of certain macromolecules to the surface of a mucous tissue is not well understood yet. This mechanism is generally divided into two steps: the contact stage and the consolidation stage. The first stage is characterized by the contact between the mucoadhesive and the mucus membrane, with spreading and swelling of the formulation, initiating its deep contact with the mucus layer. In the consolidation step, the mucoadhesive materials are activated by the presence of moisture that plasticizes the system, allowing the mucoadhesive molecules to break free and to link up by weak van der Waals and hydrogen bonds. Mucoadhesion remains a complex process and numerous theories have been proposed to explain the mechanisms involved. These theories include mechanical interlocking, electrostatic, diffusion interpenetration, adsorption or fracture processes (Boddupalli B.M. et al., J Adv Pharm Technol Res. 2010 Oct-Dec; 1(4): 381-387).

The oral delivery systems are not a peculiarity confined to mammals alone. For example, data on this subject are reported in literature in fish. Oral release systems, suitable to protect the antigen in the stomach of fish and to allow uptake at intestinal level, where it can be processed by the competent cells, are capable of evoking a protective immune response (Schep L. J., et al. 1999 ref. cit.). With reference to the local immune response, it has been observed that the second segment of the terminal intestine of fish is able to absorb soluble or particulate antigens (Schep L. J., et al. 1999 ref. cit.). Furthermore, specific antibodies have been found in various orally vaccinated fish species such as trout, bass, catfish and flounders (Ainsworth A. J. et al. 1995 J. Fish Disease, 18(5): 397-409).

The development of large farms in the field of aquaculture today requires farmers to practice a highly efficient medical care because of its economic impact. Several options are available to them but more often, they prefer to be safe than cure and therefore they prefer to use methods such as vaccination, when available. Indeed, prevention can limit the use of major treatments that are performed by the dissemination of antibiotics in the culture medium of fish and shellfish. However, increased use of antibiotics inevitably leads to an increase of bacterial resistance to these antibiotics, which eventually will mean that many of them will be ineffective. In addition, in the case of viral diseases, prevention through immunization remains the only effective therapy.

Most of the existing vaccines are administered to aquatic animals by injection, which is traumatic, inconvenient, time consuming, expensive, has a number of side effects, and may fail to induce an appropriate immunogenic response in mucosal tissues. In immersion vaccination, there are two application methods: dip and bath. In dip vaccination, fish are manipulated and are immersed for a very short duration, usually 30 seconds, in a highly concentrated vaccine solution, usually 1 part vaccine product to 9 parts water. With bath vaccination, fish are exposed for a longer period, usually one to several hours, in a lower concentration of vaccine. The immersion vaccines necessitate huge amounts of medicinal products.

Thus, a method and system for delivery that avoids these disadvantages would be advantageous.

Oral immunization would be an ideal alternative, since this is less time consuming and labour intensive, less stressful for fish and allows mass vaccination of fish of any size.

Oral vaccination with antigen, wherein the vaccine is either mixed with the feed, coated on top of the feed (topdressed) or bio-encapsulated (incorporationg of nutrients or medicines into living organism that can then be fed to the target animal) are known in the art. When antigens are to be incorporated in feed, the heat sensitivity of the antigen has to be considered. When vaccines are used as top dressing in feed, a coating agent is usually applied, either to prevent leaching of the antigen from the pellets or to prevent breakdown of the antigen in the acidic environment of the fish stomach.

Bio-encapsulation can be used where fish fry are to be vaccinated. For example, live feed, such as Artemia nauplii, copepods or rotifers, are incubated in a vaccine suspension after which they are fed to the fry. Since these live organisms are non-selective filter feeders, they will accumulate the antigen in their digestive tract and as such, transform themselves into living microcapsules.

In order to obtain efficient systems for intestinal absorption following oral administration, it is important that:
i) the pharmaceutically active agents are not significantly degraded in an acid environment at gastric level; the composition comprising said agents has to protect them from degradation and there must be no significant release in an acid environment, thus allowing said substances to reach the intestine;
ii) the outer surface of said composition has good bioadhesive capacities so that they can adhere to the intestinal mucosa and release the formulated pharmaceutically active substances in a controlled way for the purpose of inducing an immune response;
iii) said composition produces an efficient immune response;
iv) said composition remains stable during storage.

It is also important for the oral carrier system as a whole to be easily administered and to be easily produced at industrial level and for it to have moderate costs.

Some well-known antigen delivery systems are those derived from the linear polymeric esters of lactic acid and glycolic acid (i.e., poly DL-lactide-co-glycolide, PLGA, reviewed by Wu (Wu, 2004). In such systems, immunogenic subunit vaccine components have been captured in poly-acrylate and poly-glycolide/lactide beads or liposome-like vesicles through processes utilizing volatile organic solvents such as dichloromethane or chloroform. The solvents are used to form emulsions of polymer solutions or dried lipid films. Encapsulation of antigens into PLGA microcapsules affords a number of advantages including rapid degradation by hydrolysis and subsequent penetration of the Peyer's Patches (concentrated sites of lymphocytic tissue in the intestinal mucosa of higher vertebrates, but not in fish). A major disadvantage of PLGA microcapsules is the requisite use of organic solvents. Contact with organic solvents can inactivate or reduce the efficacy of the vaccine by altering the immunogenicity of surface proteins critical to induction of humoral or cellular immune responses. Additionally, poly-acrylate and poly-glycolide/lactide processes typically result in microbeads with extremely low immunogen or antigen capture efficiency.

Polymer microspheres and lamellar particles (e.g., liposomes) have been employed for the improved parenteral and mucosal administration of antigens. Because vaccines themselves may not be efficiently recognized and taken up by mucosal lymphocytes, they typically need to be co-administered with penetration enhancers or adjuvants. Different classes of polymer mixtures are known for potential use as mucoadhesives (Malik et al., 2007). These include synthetic polymers such as poly (acrylic acid) (PAA), hydroxypropyl methylcellulose and poly(methylacrylate) derivatives, as well as naturally occurring polymers such as hyaluronic acid and chitosan.

Chitosan has also been used for a variety of applications as a biomaterial for tissue engineering, wound healing, and as an excipient for drug delivery (Chopra et al., 2006; Dang and Leong, 2006). Chitosan has occasionally been tested as an adjuvant for mucosal application (Kim et al., 2007), but it is typically applied directly to a mucosal surface such as intranasal application in order to obtain IgA response in the nasopharyngeal mucosa of terrestrial animals (Kang et al., 2007). However, the use of chitosan in vaccine delivery remains limited due to its limited capacity for controlling drug release for oral dosage forms (fast dilution in the stomach). Chitosan also has the additional disadvantage of a low mechanical strength and solubility.

WO2005/013941 discloses double-layer microcapsules as carriers for the oral administration of biologically active substances that can be used for oral vaccination in the veterinary field. Such double-layer microcapsules are constituted by an outer layer of chitosan and an inner layer of alginate and a divalent ion whose functions are to be both a gelification agent of the alginate and a stabilizer for the double-layer microcapsules. Said microcapsules demonstrated to induce stimulation of the immune responses, measured by the quantity of IgM released, in a mouse model. The generation process of the microcapsules involves emulsification technique (water-in-oil (W/O) system) as well as several washing with isopropanol for purification, which may not be desirable from a manufacturing, security and environement standpoint. Furthermore, the practice demonstrated that such a technique allows to produce a few grams/day of micro-particles, with high production costs.

WO2015/084594 describes compositions for oral delivery of bioactive agent including particles each of which includes a bioactive agent dispersed in oil droplets, the oil droplets being dispersed in a matrix including an enteric coating polymer, wherein the particles each further include a mucoadhesive polymer.

US2009/238845 describes a bioadhesive delivery system comprising a composition including a cationic polysaccharide, a neutral polysaccharide in combination with a pharmaceutically active agent, such as an immunogenic agent.

It is an object of the present invention to overcome or ameliorate at least one of the disadvantages or weaknesses of the prior art, or to provide a useful alternative.

### Definitions

"About" or "approximately," when used in connection with a measurable numerical variable, refers to the indicated value of the variable and to all values of the variable that are within the experimental error of the indicated value (e.g., within the 95% confidence interval for the mean), or within 10%> of the indicated value, whichever is greater. For example, if "about" is used in reference to time intervals in weeks, "about 3 weeks" is 17 to 25 days, and "about 2 to about 4 weeks" is 10 to 40 days.

"Adjuvant", as used herein, refers to any substance which serves as a nonspecific stimulator of the immune response. See below for a further description of adjuvants.

"Amino acid", as used herein, refers to naturally-occurring and synthetic amino acids, as well as amino acid analogs and amino acid mimetics that function in a manner similar to naturally- occurring amino acids. Naturally-occurring amino acids are those encoded by the genetic code, as well as those amino acids that are later modified, for example, hydro xyproline, carboxyglutamate, and O-phosphoserine. Stereoisomers (e.g., D-amino acids) of the twenty conventional amino acids, unnatural amino acids such as a and a-disubstituted amino acids, N-alkyl amino acids, and other unconventional amino acids, may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids. Amino acids may be referred to herein by either their commonly known three-letter symbols or their one-letter symbols recommended by the ILTPAC-ILTB Biochemical Nomenclature Commission.

"Antibody", as used herein, is any polypeptide comprising an antigen-binding site regardless of the source, method of production, or other characteristics. It refers to an immunoglobulin molecule or a fragment thereof that specifically binds to an antigen as the result of an immune response to that antigen. Immunoglobulins are serum proteins composed of "light" and "heavy" polypeptide chains having "constant" and "variable" regions and are divided into classes (e.g., IgA, IgD, IgE, IgG, and IgM) based on the composition of the constant regions. In mammals and birds, the immunoglobulin M (IgM), IgG and IgY isotypes have a predominant role in systemic responses, whereas IgA is the key participant in mucosal surfaces. Teleost fish are the most primitive bony vertebrates that contain immunoglobulins and, in contrast to mammals and birds, serum IgM in most teleosts is expressed as a tetramer, although IgM monomers have been described. In addition teleosts are devoid of IgA or a functional equivalent of IgA although recent research have added two new immunoglobulin molecules, IgD and IgT/IgZ, with IgT being reported as an immunoglobulin specialized in gut mucosal immunity. An antibody that is "specific" for a given antigen indicates that the variable regions of the antibody recognize and bind a specific antigen exclusively. The term includes, but is not limited to: a polyclonal antibody, a monoclonal antibody, a monospecific antibody, polyspecific antibody, humanized antibody, a tetrameric antibody, a tetravalent antibody, a multispecific antibody, a single chain antibody, a domain- specific antibody, a single domain antibody, a domain-deleted antibody, a fusion protein, an ScFc fusion protein, a single-chain antibody, chimeric antibody, synthetic antibody, recombinant antibody, hybrid antibody, mutated antibody, and CDR-grafted antibodies. Antibodies can be intact immunoglobulins derived from natural sources or from recombinant sources, or can be immunoreactive portions of intact immunoglobulins. An "antibody" can be converted to an antigen-binding protein, which includes but is not limited to antibody fragments which include but are not limited to: Fab, F(ab') 2, an Fab' fragment, an Fv fragment, a single-chain Fv (ScFv) fragment, an Fd fragment, a dAb fragment, diabodies, a CDR3 peptide, a constrained FR3-CDR3-FR4 peptide, a nanobody, a bivalent nanobody, a small modular immunopharmaceutical (SMIPs), and a minibody and any of above mentioned fragments and their chemically or genetically manipulated counterparts, as well as other antibody fragments that retain antigen-binding function. Typically, such fragments would comprise an antigen-binding domain. As will be recognized by those of skill in the art, any of such molecules may be engineered (for example "germlined") to decrease its immunogenicity, increase its affinity, alter its specificity, or for other purposes.

"Antigen" or "immunogen", as used herein, refers to a molecule that contains one or more epitopes (linear, conformational or both) that upon exposure to a subject will induce an immune response that is specific for that antigen. An epitope is the specific site of the antigen which binds to a T-cell receptor or specific antibody, and typically comprises about 3 amino acid residues to about 20 amino acid residues. The term antigen refers to subunit antigens, antigens separate and discrete from a whole organism with which the antigen is associated in nature as well as whole killed, attenuated or inactivated bacteria, viruses, fungi, parasites or other microbes. The term antigen also refers to antibodies, such as anti-idiotype antibodies or fragments thereof, and to synthetic peptide mimotopes that can mimic an antigen or antigenic determinant (epitope). The term antigen also refers to an oligonucleotide or polynucleotide that expresses an antigen or antigenic determinant *in vivo*, such as in DNA immunization applications.

"Antigenicity", as used herein, refers to the capability of a protein or polypeptide to be immunospecifically bound by an antibody raised against the protein or polypeptide.

"Bioadhesive substance", as used herein, is broadly defined as a material that is capable of being bound to a biological membrane and retained on that membrane for an extended period of time. The term "bioadhesion" relates to the attachment of a material to a biological substrate such as a biological membrane. In the present invention, the terms "bioadhesion" and "mucoadhesion", and "bioadhesive" and "mucoadhesive" are used indifferently. A "bioadhesive delivery composition" is defined as a composition that results in the delivery of a pharmaceutically active agent, preferably an immunogen, to the desired location in the gut associated lymphoid tissue (GALT) of the intestinal mucosa. A bioadhesive or mucoadhesive polymer is a polymer able to strong interaction with mucosa, preferably the intestinal mucosa.

"Booster", as used herein, is broadly defined as a dose that is an extra administration of a vaccine after an earlier (prime) dose. "Boostering" or "Booster vaccination" relate to the administration of the booster dose to the subject. After initial immunization, that do not nescessarily occurs at the same dosage or at the same mode of administration, a boostering leads to a re-exposure to the immunizing antigen. It is intended to increase immunity against that antigen back to protective levels, after memory against that antigen has declined through time.

"Buffer" means a chemical system that prevents change in the concentration of another chemical substance. Proton donor and acceptor systems serve as buffers, preventing marked changes in hydrogen ion concentration (pH). A further example of a buffer is a solution containing a mixture of a weak acid and its salt (conjugate base), or a weak base and its salt (conjugate acid).

The term "cell line" or "host cell", as used herein, means a prokaryotic or eukaryotic cell in which a virus can replicate or be maintained. The term "culture", as used herein, means a population of cells or microorganisms growing in the absence of other species or types.

"Dose" refers to a composition, in particular a vaccine or immunogenic composition given to a subject. A "first dose" or "priming dose" refers to the dose of such a composition given on Day 0. A "second dose" or a "third dose" or an "annual dose" refers to an amount of such composition given subsequent to the first dose, which can be, but is not required to be, the same vaccine or immunogenic composition as the first dose.

An "epitope" is the specific site of the antigen which binds to a T-cell receptor or specific antibody, and typically comprises from about 3 amino acid residues to about 20 amino acid residues. "Excipient", as used herein, refers to a non-reactive carrier component of a vaccine or immunogenic composition that is not an antigen. Preferred excipients are those known in the art for immersion vaccines.

The term "fish" refers to both cold and warm water fish that can be treated by the vaccine compositions of the present invention. Fish that may be treated by the method of the invention include any fish that is susceptible to infection and disease caused by the particular organism or pathogen. The fish may be a marine or salt-water fish. Examples of suitable fish for the method of invention include salmonids (Oncorhynchus sp. and Salmo sp.), American, European, and Japanese eels (Anguilla sp.), tilapia (Oreochromis sp.), striped bass and hybrid-striped bass (Morone chrysops and M. saxatilis), flounders (Seriola sp.), seabream (Spams sp.), sea perch (Lates calcarifer), the estuarine grouper (Epinephelus tawine), walleye (Stitzostedion vitreum), channel catfish (Ictalurus punctutus), centrachids (such as largemouth bass, Micropterus salmoides), brown bullheads (Nebulosus sp.), fat head minnows (Pimephales promelas), golden shiners (Netemigonus crysoleucas), carp (Cyprinus carpio), all species of tuna, aquarium fish species such as black mollies (Poecilia sphenops), platies (Xiphosphorus maculatus), sea lamprey (Petromyzon marinus), tench (Tinea tinea), crucian carp (Carassius carassius), goldfish (Carassius auratus), sweetfish (Plecoglossus altivelis), freshwater minnows (Zacco platypus), as well as perch (Perca fluviatilis), roach (Rutilus rutilus) and sea bass (Serranidae sp.). In a preferred embodiment, the fish are used in aquaculture.

"Fragment" refers to a truncated portion of a protein or gene. "Functional fragment" and "biologically active fragment" refer to a fragment that retains the biological properties of the full length protein or gene.

As used herein, the term "freeze-drying" refers to the drying of a deep-frozen material under high vacuum by freezing out the solvent (ie. water) and then evaporating it in the frozen state.

"Homology" or "percent homology" refers to the percentage of nucleotide or amino acid residues in the candidate sequence that are identical or similar with the residues in the comparator sequence(s) after aligning the sequences and introducing gaps, if necessary, to achieve the maximum percent sequence homology, and also considering any conservative substitutions as part of the sequence homology. In a preferred aspect, polynucleotides or amino acid sequences provided herein have greater than about 60% sequence homology, greater than about 70% sequence homology, greater than about 80% sequence homology, greater than about 90% sequence homology, greater than about 95% sequence homology, greater than about 96% sequence homology, greater than about 97% sequence homology, greater than about 98% sequence homology, or greater than about 99% sequence homology.

"Identity" or "percent identity" refers to the percentage of nucleotides or amino acids in the candidate sequence that are identical with the residues in the comparator sequence after aligning both sequences and introducing gaps, if necessary, to achieve the maximum percent sequence identity, and not considering any conservative substitutions as part of the sequence identity. In a preferred aspect, polynucleotides or amino acid sequences provided herein have, to the reference sequence (e.g. SEQ ID NO: x, y, or z) greater than about 60% sequence identity, greater than about 70% sequence identity, greater than about 80% sequence identity, greater than about 90% sequence identity, greater than about 95% sequence identity, greater than about 96% sequence identity, greater than about 97% sequence identity, greater than about 98% sequence identity, or greater than about 99% sequence identity.

In the present invention, the term "immunostimulant" related to a "compound or pharmaceutical composition" is defined herein in a broad sense to refer to any type of biological agent denote a compound, or a pharmaceutical composition, capable of stimulating an immune response, especially capable of increasing a nonspecific immune response.

"Immune response", as used herein, in a subject refers to the development of a humoral immune response, a cellular immune response, or a humoral and a cellular immune response to an antigen. A "humoral immune response" refers to one that is at least in part mediated by antibodies. A "cellular immune response" is one mediated by T-lymphocytes or other white blood cells or both, and includes the production of cytokines, chemokines and similar molecules produced by activated T-cells, white blood cells, or both. Immune responses can be determined using standard immunoassays and neutralization assays, which are known in the art.

"Immunogenicity", as used herein, refers to the capability of a protein or polypeptide to elicit an immune response directed against a bacteria or virus that causes the identified disease.

An "immunogenic composition" is a preparation containing an immunogen, including, e.g., a protein, a peptide, a whole cell, inactivated, subunit or attenuated virus, or a polysaccharide, or combination thereof, administered to stimulate the recipient's humoral and cellular immune systems to one or more of the antigens present in the immunogenic composition.

"Immunization" is the process of administering an immunogenic composition and stimulating an immune or immunogenic response to an antigen in a host. Preferred hosts are fish, such as fish used in aquacultre. Preferably, the immunogenic composition is a vaccine.

"Immunologically protective amount", as used herein, is an amount of an antigen effective to induce an immunogenic response in the recipient that is adequate to prevent or ameliorate signs or symptoms of disease, including adverse health effects or complications thereof. Either humoral immunity or cell-mediated immunity or both can be induced. The immunogenic response of an animal to a composition can be evaluated, e.g., indirectly through measurement of antibody titers, lymphocyte proliferation assays, or directly through monitoring signs and symptoms after challenge with wild type strain. The protective immunity conferred by a composition or vaccine can be evaluated by measuring, e.g., reduction of shed of challenge organisms, reduction in clinical signs such as mortality, morbidity, temperature, and overall physical condition, health and performance of the subject. The immune response can comprise, without limitation, induction of cellular and/or humoral immunity. The amount of a composition or vaccine that is therapeutically effective can vary, depending on the particular organism used, or the condition of the animal being treated or vaccinated, and can be determined by a veterinarian.

The term "isolated" refers to a substance that is either in substantially pure form, for example, greater than about 95% purity; or purified, enriched or removed in some way from its natural environment. Reference to "isolated" indicates an agent, such as a protein that is removed from its natural environment, such as from a host animal/fish, in a growth media, recombinantly produced, or purified from a whole cell bacterial preparation and may subsequently be added back to a composition comprising other actives or antigens. The term "isolated" encompasses immunogens that are in solution with other agents/diluents/excipients/adjuvants/proteins.

"Medicinal agent" refers to any agent which is useful in the prevention, cure, or improvement of a medical condition, or the prevention of some physiological condition or occurrence.

"Mixture" as used herein refers to a composition or a formulation of at least one component. Reference to a "liquid mixture" indicates that the composition or formulation is in liquid form, in opposition to solid state or dry state or gaz. The liquid mixture is fluid, flowable and is either a solution, a suspension, a gel (hydrogel), an emulsion.

The term "mucoadhesive" is defined as a material in which an adhesive bonding is established between a material and the mucosa/mucus/mucin of a biological membrane. The term "mucoadhesive substance" is in accordance with the generally accepted terminology and is used synonymously with the term "bioadhesive substance".

"Monoclonal antibody", as used herein, refers to antibodies produced by a single line of hybridoma cells, all directed towards one epitope on a particular antigen. The antigen used to make the monoclonal antibody can be provided as an isolated protein of the pathogen or the whole pathogen. A "hybridoma" is a clonal cell line that consists of hybrid cells formed by the fusion of a myeloma cell and a specific antibody-producing cell. In general, monoclonal antibodies are of mouse origin. However, monoclonal antibody also refers to a clonal population of an antibody made against a particular epitope of an antigen produced by phage display technology, or method that is equivalent to phage display, or hybrid cells of non-mouse origin. "Parenteral administration", as used herein, refers to the introduction of a substance, such as a composition or vaccine, into a subject's body through or by way of a route that does not include the digestive tract. Parenteral administration includes subcutaneous, intramuscular, intraarterial, and intravenous administration. For the purposes of this disclosure, parenteral administration excludes administration routes that primarily involve transport of the substance through mucosal tissue in the mouth, nose, trachea, and lungs. The term "pathogen" or "pathogenic microorganism", as used herein, means a microorganism - for example *Flavobacterium columnare* (F. columnare), *Flavobacterium psychrophilum* (F. psychrophilum), *Tenacibaculum maritimum* (T. maritimum), and infectious pancreatic necrosis virus (IPNV) - which is capable of inducing or causing a disease, illness, or abnormal state in its host animal, preferably a disease affecting fish.

"Oral administration", as used herein, refers to the introduction of a substance, such as a vaccine or other composition, into a subject's body through oral, buccal, enteral or intragastric administration.

"Pharmaceutically acceptable" refers to substances which, within the scope of sound medical judgment, are suitable for use in contact with the tissues of subjects without undue toxicity, irritation, allergic response, and the like, commensurate with a reasonable benefit-to-risk ratio, and effective for their intended use.

"Polyclonal antibody", as used herein, refers to a mixed population of antibodies made against a particular pathogen or antigen. In general, the population contains a variety of antibody groups, each group directed towards a particular epitope of the pathogen or antigen. To make polyclonal antibodies, the whole pathogen, or an isolated antigen, is introduced by inoculation or infection into a host, which induces the host to make antibodies against the pathogen or antigen.

The term "polynucleotide", as used herein, means an organic polymer molecule composed of nucleotide monomers covalently bonded in a chain. DNA (deoxyribonucleic acid) and RNA (ribonucleic acid) are examples of polynucleotides with distinct biological function.

The term "polypeptide", as used herein, means an organic polymer molecule composed of two or more amino acids bonded in a chain.

"Preventing infection", as used herein, means to prevent or inhibit the replication of the bacteria or virus which cause the identified disease, to inhibit transmission of the bacteria or virus, to prevent the bacteria or virus from establishing itself in its host, or to alleviate the symptoms of the disease caused by infection. The treatment is considered therapeutic if there is a reduction in bacterial or viral load.

"Protection", "protecting", "protective immunity", and the like, as used herein with respect to a vaccine or other composition, means that the vaccine or composition prevents or reduces the symptoms of the disease caused by the organism from which the antigen(s) used in the vaccine or composition is derived. The terms "protection", "protecting", and the like, also mean that the vaccine or composition can be used to "treat" the disease, or one or more symptoms of the disease that already exists in a subject.

"Relative Percent Survival" (RPS) can be defined or computed as follows: RPS= f 1- (% vaccinate mortality / % control mortality)} × 100.

"Specific immunogenic fragment", as used herein, refers to a portion of a sequence that is recognizable by an antibody or T cell specific for that sequence.

"Subject", as used herein, refers to any animal having an immune system, preferably fish.

"Substantially identical", as used herein, refers to a degree of sequence identity of at least about 90%, at least about 95%, at least about 96%, at least about 97%, at least about 98%, or at least about 99%.

"Subunit vaccine", and "subunit composition", as used herein, refers to a type of vaccine or composition that includes one or more antigens -but not all antigens-which are derived from or homologous to, antigens from a pathogen of interest, such as a virus, bacterium, parasite, fungus, or fungal-like organism. Such a composition or vaccine is substantially free of intact pathogen cells or pathogenic particles, or the lysate of such cells or particles. Thus, a subunit vaccine or subunit composition can be prepared from at least partially purified, or substantially purified, immunogenic polypeptides from the pathogen or their analogs. Methods of obtaining an antigen or antigens in the subunit vaccine or subunit composition include standard purification techniques, recombinant production, or chemical synthesis. A "subunit vaccine" or "subunit composition" thus refers to a vaccine or composition consisting of a defined antigenic component or components of a virus, bacterium, or other immunogen. The subunit component of the present invention can be recombinantly produced and can comprise IPNV VP2 antigen and/or F. psychrophilum antigen.

"Therapeutic agent", as used herein, refers to any molecule, compound, virus or treatment, preferably a virus attenuated or killed, or subunit or compound, that assists in the treatment of a viral, bacterial, parasitic or fungal infection, disease or condition caused thereby.

"Therapeutically effective amount", as used herein, refers to an amount of an antigen or vaccine or composition that would induce an immune response in a subject (e.g., fish) receiving the antigen or vaccine or composition which is adequate to prevent or ameliorate signs or symptoms of disease, including adverse health effects or complications thereof, caused by infection with a pathogen, such as a virus, bacterium, parasite, fungus, or fungal-like organism. Humoral immunity or cell -mediated immunity, or both humoral and cell-mediated immunity, can be induced. The immunogenic response of an animal to an antigen, vaccine, or composition can be evaluated indirectly through measurement of antibody titers, lymphocyte proliferation assays, or directly through monitoring signs and symptoms after challenge with the wild type strain. The protective immunity conferred by a vaccine or composition can be evaluated by measuring reduction of challenge organism shed, and/or reduction in clinical signs, such as mortality, morbidity, temperature, and overall physical condition, health, and performance of the subject. The amount of a vaccine or composition that is therapeutically effective can vary, depending on the particular immunogen used, or the condition of the subject, and can be determined by one skilled in the art.

"Treat" or "treating", as used herein, refers to reversing, alleviating, inhibiting the progress of, or preventing a disorder, condition or disease to which such term applies, or to preventing one or more symptoms of such disorder, condition or disease.

"Vaccine" or "vaccine composition," as used herein, refers to an immunogenic composition selected from a virus or bacteria. Administration of the vaccine to a subject results in an immune response. The vaccine can be introduced directly into the subject by any known route of administration, preferably via the oral route. The terms mean a composition which prevents or reduces an infection, or which prevents or reduces one or more signs or symptoms of infection. The protective effects of a vaccine composition against a pathogen are normally achieved by inducing in the subject an immune response. Generally speaking, abolished or reduced incidences of infection, amelioration of the signs or symptoms, or accelerated elimination of the microorganism from the infected subjects are indicative of the protective effects of a vaccine composition.

The present disclosure is based upon the unexpected discovery that inclusion of antigens in oral vaccines according to the invention results in substantially improved efficacy, efficiency and safety. That is, when administered to a fish prior to pathogenic challenge, the composition prevents the onset of disease, and does not result in adverse side-effects.

One purpose of the present invention is to develop a new solid system for oral administration of pharmaceutically active agent, suitable to determine absorption essentially at intestinal level, in natural polysaccharide composition that has the characteristics of:
i) comprising at least one pharmaceutically active agent so that it is protected from degradation in an acid environment at gastric level;
ii) ensuring the stability, efficacy, and safety of said at least one pharmaceutically active agent during its shelf life;
iii) allowing release of said at least one pharmaceutically active agent at intestinal level;
iv) having good bioadhesive capacities so that it adheres to the intestinal mucosa and releases said at least one pharmaceutically active agent in a controlled way;
v) enabling a cost-effective and simple industrial manufacturing with high rates of production.

Moreover, the new oral carrier system according to the invention must be able to comprise one or more pharmaceutically active agents so that an adequate biological response is obtained from their release.

Thanks to the selection of a specific combination of biocompatible components of the carrier, in adequate relative proportions and thanks to the effective control of the size of the particles formed therefrom, the present invention overcomes the shortcomings of the above-discussed drug delivery systems. The present invention discloses a composition designed for an improved oral delivery of a pharmaceutically active agent, such as a primary and or booster vaccination, that can be used for livestock animals including mammals and fishes. The good mucoadhesive properties of compositions of the present invention provide a successful method of transmucosal drug delivery, especially for lower vertebrates with less developed digestive system and no Peyer's Patches, such as fish.

One aspect of the present invention provides for a composition for oral administration to an animal for intestinal delivery of an immunogenic agent, said composition being in the form of microparticles and comprising:a) at least one mucoadhesive cationic polymer that is chitosan;b) at least two anionic components, wherein at least one of the two anionic components being dextran sulfate;c) at least one immunogenic agent; for use in the oral vaccination or boostering of an animal against a pathogen.

Surprisingly, said oral bioadhesive delivery composition results in a similar or better immunologic induction than parenteral administration of the pharmaceutically active agent and than existing fish oral vaccines. The selection of at least two anionic components combined to the cationic polymer chitosan enables to increase antigen protection of the harsh conditions of the gastrointestinal tract, to increase encapsulation efficiency and/or antigen release.

Bioadhesive substances, also denoted mucoadhesive substances, are generally known to be materials that are capable of being bound to a biological membrane and possibly retained on that membrane for an extended period of time. Compared with conventional release systems, bioadhesive systems have the following advantages:
i) a bioadhesive system localizes a biological active ingredient in a particular region, thereby improving and enhancing the bioavailability for active ingredients which may have poor bioavailability by themselves,
ii) a bioadhesive system leads to a relatively strong interaction between a bioadhesive substance and a mucosa, such an interaction contributes to an increasing contact time between the release system and the tissue in question and permits localization of the active released from the release system to a specific site,
iii) a bioadhesive release system may provide controlled release and prolongs delivery of biological active ingredients in almost any non-parenteral route,
iv) a bioadhesive release system can be localized on a specific site with the purpose of local therapy,
v) a bioadhesive release system can be targeted to specific diseased tissues, and
vi) a bioadhesive release system is useful when conventional approaches are unsuitable, such as for certain biological active ingredients which are not adequately absorbed.

The oral bioadhesive delivery composition according to the invention allows the release of the pharmaceutically active agent at the site of action (i.e., the Gut Associated Lymphoid Tissue; GALT) along the foregut and hindgut of the animal. It further provides protection of the pharmaceutical active agent during transit through the stomach of the animal and then provides a gradual dissolution, corresponding to the hindgut transit time of about 2 hours, and permits reproducible release of the active agent therein.

More specifically, the oral bioadhesive delivery composition according to the present invention is a composition for oral administration to an animal for intestinal delivery of an immunogenic agent, said composition comprising:
a) at least one mucoadhesive cationic polymer that is chitosan;
b) at least two anionic components, wherein at least one of the two anionic components being dextran sulfate;
c) at least one immunogenic agent;

for use in the oral vaccination or boostering of an animal against a pathogen;
   - preferably, the at least one other anionic component is selected in the group consisting in alginate and fucoidan; more preferably, said at least one other anionic components is alginate;
   - according to a specific embodiment, the composition for oral administration to an animal for intestinal delivery of an immunogenic agent according to the invention, further includes at least one divalent ion;
   - according to a specific embodiment, the animal is a fish and the pathogen is causing the Salmon Rickettsial Syndrome (SRS);
the present invention also relates to:
   - an animal feed comprising the composition for oral administration to an animal for intestinal delivery of an immunogenic agent according to the invention;
   - a method of preparing an animal feed consisting in blending the composition for oral administration to an animal for intestinal delivery of a pharmaceutically active agent according to the invention with an oil and top-coating feed pellets.

According to a general disclosure that is not fully covered by the claims,
the present disclosure relates to oral bioadhesive delivery compositions that include composition for oral administration to an animal for intestinal delivery of an immunogenic agent, said composition comprising:
a) at least one mucoadhesive cationic polymer,
b) at least two anionic components,
c) at least one pharmaceutically active agent,
d) optionally divalent ions,
e) optionally, at least one lyophilisation agent, and
f) optionally, at least one immunostimulant.

The cationic mucoadhesive polymer used in the present invention is chitosan; other frequently used cationic mucoadhesive polymers include aminodextran, diethylaminoethyl (DEAE)-dextran and polylysine, which - due to the presence of amine groups - interact with anionic substructures of sialic acid residues in the mucus layer (Nallathambi and Gopal, 2013). Chitosan is quickly hydrated in low pH environments, e.g. gastric acid pH, does not swell and does not exhibit greater mucoadhesion in pH above 6.5. Some cationic polymers are likely to demonstrate superior mucoadhesive properties, especially in a neutral or slightly alkaline medium. Additionally, some cationic high-molecular-weight polymers, such as chitosan, have shown to possess good adhesive properties. There is no significant literature about the influence of the charge of the membrane on the mucoadhesion, but the pH of the membrane affects the mucoadhesion as it can influence the ionized or un-ionized forms of the polymers.

The composition of the invention is in the form of microparticles.

At least one anionic component is dextrane sulfate and the at least one other anionic component is preferably selected in the group consisting in alginate and fucoidan. The microparticles of the composition may comprise one or more additional other anionic components.

### Chitosan

Chitosan is a linear cationic polysaccharide which is gelled or crosslinked in the presence of anions, such as citrate, phosphate or sulfate. Chitosan has been shown to possess useful properties such as non-toxicity, high biocompatibility and non-antigenicity. While chitosan is itself largely insoluble in water, solubility markedly increases if the pH is shifted towards the acid condition.

Chitosan can be obtained through the deacetylation of chitin, the major compound of exoskeletons in crustaceans. Chitosan is composed of β-1,4-linked glucosamine (deacetylated units) and N-acetyl-D-glucosamine (acetylated units). The deacetylation may be more or less important and therefore form chitosans having different properties according to their degree of acetylation. Chitosan is a mucopolysaccharide closely related to cellulose, it exhibits chemical properties that are determined by the molecular weight, degree of deacetylation, and viscosity. Chitosan can form microparticles and nanoparticles that can encapsulate large amounts of antigens (Van der Lubben et al., 2001; Davis, 2006). In the acidic environment of the stomach, chitosan retains its positive charges that hold the particle together. Furthermore, chitosan can establish ionic, hydrogen or hydrophobe linkages with the negatively charged mucine, conferring it mucoadhesive properties useful for oral administration.

Preferably, chitosan according to the invention has a deacetylation degree at least/from about 70% to about 95%, preferably from about 80% to about 95% and more preferably from about 85 to about 95%, and even more preferably from about 90% to 95%, most preferably 95%. Most preferably, chitosan of the invention has a deacytalation degree of at least 85%, at least 90%, 91%, 92%, 93%, 94% and even most preferably at least 95%.

Preferably, chitosan has a molecular weight of between about 10 kDa and 1000 kDa, corresponding to low molecular weight (20-300 mPa.s) and medium molecular weight (with a range of viscosities between about 20mPa.s and about 800 mPa.s) and more preferably with a viscosity between about 50 mPa.s and about 200 mPa.s, Indeed, the viscosity of the chitosan is linked to its molecular weight and it has been observed that it is preferred that the viscosity not exceed 1000 mPa.s in order to avoid possible problems in the manufacturing process.

In another embodiment, chitosan according to the invention can be a mix of chitosan having different molecular weights.

The chitosan concentration in the initial solution of chitosan is preferably ranging from about 0,2 to about 5%, preferably from about 0.5% to about 3%, even more preferably from about 1% to about 1,5%.

Most preferably, the chitosan concentration in the initial solution of chitosan according to the invention is about 1%.

Chitosan is soluble in water only in acidic medium. Then in order to solubilized it, 1% of acetic acid is introduced in one part of total volume of water. 1% of weighted chitosan is dissolved under stirring with a propeller or a deflocculator. Then after dissolution, pH of the solution is adjuted at 5,5 or 5,6. For it, 10N NaOH solution is introduce drop by drop in the solution under stirring with a propeller or a deflocculator. pH control is carried out during introduction of 10N NaOH solution to reach the target pH.

The at least two anionic components are preferably anionic polymers.

Such anionic polymers include dextran sulfate, alginate, fucoidan, Tripolyphosphate (TPP), carboxymethylcellulose sodium, poly(acrylic acid) - based polymer (PAA or Carbomer) or natural polymer as pectin or xanthan gum.

At least one anionic component is dextrane sulfate and the at least one other anionic component is preferably selected in the group consisting in alginate and fucoidan, the microparticles of the composition may comprise one or more additional other anionic components.

### Dextran

Dextrans are polysaccharides with molecular weights ≥1000 Dalton, which have a linear backbone of α-linked D-glucopyranosyl repeating units. Three classes of dextrans can be differentiated by their structural features:
Class 1 dextrans contain the α(1→6)-linked D-glucopyranosyl backbone modified with small side chains of D-glucose branches with α(1→2), α(1→3), and α(1→4)-linkage. The class 1 dextrans vary in their molecular weight, spatial arrangement, type and degree of branching, and length of branch chains, depending on the microbial producing strains and cultivation conditions. Isomaltose and isomaltotriose are oligosaccharides with the class 1 dextran backbone structure.
Class 2 dextrans (alternans) contain a backbone structure of alternating α(1→3) and α(1→6)-linked D-glucopyranosyl units with α(1→3)-linked branches.
Class 3 dextrans (mutans) have a backbone structure of consecutive α(1→3)-linked D-glucopyranosyl units with α(1→6)-linked branches. One and two-dimensional NMR spectroscopy techniques have been utilized for the structural analysis of dextrans.

The physical and chemical properties of purified dextrans vary depending on the microbial strains from which they are produced and by the production method. Dextrans have high water solubility and the solutions behave as Newtonian fluids. Solution viscosity depends on concentration, temperature, and molecular weight, which have a characteristic distribution. The hydroxyl groups present in dextran offer many sites for derivatization.

Dextran is synthesized from sucrose by certain lactic acid bacteria such as Leuconostoc mesenteroides and Streptococcus mutans.

In a preferred embodiment, dextran according to the invention is dextran sulphate. Even more preferably, the dextran is dextran sulphate sodium or any other salt of dextran sulphate.

Dextran sulphate is prepared by reaction of dextran with chloro-sulphonic acid, followed by purification. Each glucose unit in the dextran chain has approximate two sulphate groups, most often located at the second and fourth carbon (C2 and C4) of the glucose unit.

Dextran sulphate presents different properties of interest for oral vaccination including mucoadhesion and interaction with the antigen. Indeed, Dextran sulfate helps in generating a strong interaction with proteins by coacervation (interaction with antigen free amines) and also acts as a mucoadhesive substance (mucus layer reduction) by fixing to membranes presenting a positive charge and being fewly adsorbed at the intestinal level. In the composition of the invention, the dextran sulphate also helps to promote the ionotropic gelation or coacervation with chitosan, it also helps to strongly interact with the antigen by coacervation (interaction with antigen free amines) and also as mucoadhesive substance (mucus layer reduction).

Preferably, dextran sulfate has a molecular weight (MW) of between 100.000 daltons (Da) and 1.000.000 Da.

High molecular weight (MW) polymers present better properties for protein coacervation and are effective stabilizers and mucoadhesive compounds. The mucoadhesive strenght is enhances when the polymers have a MW higher than 100.000 but not more than 1.000.000 MW in order to maintain the flexibility of the hydrogel of the composition.

Preferably, dextran sulfate has a MW of about between 200 000 Da and about 800 000 Da, preferably from about 300 000 Da and about 700 000 Da, preferably from about 400 000 Da and about 600 000 Da, preferably from 450 000 Da and 550 000 Da. Most preferably, dextran sulfate according to the invention has a molecular weight of 500 000 Da.

The dextran sulfate concentration in the initial mixture of ingredient (before mixing with the cationic polymer solution) is preferably ranging from about 0.1 to about 5%, preferably from about 0,3 to about 2%, and even most preferably from about 0,3 to about 1%. Most preferably, the dextran sulfate concentration in the initial mixture of ingredient is about 0,5%.

### Alginate

Alginate is a natural, linear, unbranched polysaccharide consisting of 1,4-linked β-D-mannuronic acid (M) and α-L-guluronic acid (G) monomers in varying proportions. The G/M composition depends of its source, the harvesting season and the molecular arrangement of the alginate. This non-toxic, biodegradable, naturally occurring polysaccharide is extracted from brown seaweeds and marine algae such as Laminaria hyperborea, Ascophyllum nodosum and Macrocystis pyrifera. Alginate is known for its mucoadhesive properties and is widely used in the pharmaceutical industries as it is biocompatible and non-irritant.

Without wishing to be bound by theory, it is believed that the negatively charged carboxylic acid groups of manuronic and guluronic acid units in alginate interact electrostatically with calcium ions enabling the formation of a hydrogel but also interact with the positively charged amino groups of chitosan to form a polyelectrolyte complex.

According to the present invention, alginate is preferably sodium or calcium alginate. In a preferred embodiment, sodium alginate is used; sodium alginate is soluble in water and form reticulated structure which can be cross-linked with divalent or polyvalent cations to form insoluble meshwork.

In another preferred embodiment, the alginate in the composition of the invention shows a viscosity comprised between about 4 mPa.s and 100 mPa.s (measured at 1% in water at room temperature, 20-25°C), preferably comprised between about 4 mPa.s and about 50 mPa.s , preferably comprised between about 5 mPa.s and about 40 mPa.s.

The intial alginate concentration in said composition is preferably ranging from about 0,1% to about 5%, preferably from about 0,3% to about 2%, and even most preferably from about 0,3% to about 1%.

Most preferably, the alginate concentration in said composition is about 0,5%.

### Fucoidan

Fucoidan refers to a type of polysaccharide which contains substantial percentages of L-fucose and sulfate ester groups, mainly derived from brown seaweed. Fucoidan designates a group of fucose-containing sulfated polysaccharides (FCSPs) that have a backbone built of (1→3)-linked α-1-fucopyranosyl or of alternating (1→3)- and (1→4)-linked α-1-fucopyranosyl residues, but also include sulfated galactofucans with backbones built of (1→6)-β-d-galacto- and/or (1→2)-β-d-mannopyranosyl units with fucose or fuco-oligosaccharide branching, and/or glucuronic acid, xylose or glucose substitutions.

The fucoidan concentration in the initial mixture of ingredient (before mixing with the cationic polymer solution) is preferably ranging from about 0.1 to about 5%, preferably from about 0,3 to about 2%, and even most preferably from about 0,3 to about 1%. Most preferably, the dextran sulfate concentration in the initial mixture of ingredient is about 0,5%.

### TPP

Tripolyphosphate, or sodium tripolyphosphate (TPP) is an inorganic compound having the formula Na₅P₃O₁₀. Its negatively charged phosphate molecules enable to cross-link the chitosan as the other anionic components disclosed.

The at least two anionic component according to the invention are present in respective ratio varying from 1-99 to 99-1, preferably from 10-90 to 90-10, more preferably from 30-70 to 70-30. In a preferred embodiment, the at least two anionic components are present in a ration de 60-40 to 40-60 and most referably in a ration of 48-52 to 52-48 and preferably of 50-50.

According to one embodiment, the invention relates to a specific selection of the at least two anionic polymers, said selection consisting in alginate and dextran sulfate. This embodiment is particularly advantageous compared to the existing formulations from the prior art. Indeed, chitosan and alginates are molecules that in certain conditions form pH-sensitive hydrogels. However, by theirselves these polymers cannot entrap drugs with high efficacy, which is why other polymers are needed (ionotropic gelation technique).

The alginate is a candidate of choice as an anionic polymer considering its swelling behaviour in the presence of calcium, allowing a better encapsulation of the antigen. However, hydrogels formed by alginate/calcium are not an efficient delivery system because in an acidic pH (stomach) the calcium ions will dissociate from the alginate, reducing gel strength and thus allowing the diffusion/destruction of the antigen.

The inventors demonstrated that the use of a second anionic component, such as but not limited to dextran sulfate, enabled to reinforce the network form to entrap the antigen. Dextran sulfate's sulfonates groups (SO₃⁻) are not pH-sensitive due to they are permanently charged. At pH 1, the negative sulfonates groups of the dextran sulfates can interact with the positively charged antigen or chitosan, thus maintaining matrix strength and avoiding antigen diffusion. Furthremore, the dextran sulfate is a component of choice considering its mucus layer reduction properties and its capacity to coacervate with the antigen. Coacervation is the same principle than ionotropic gelation, the association of molecules of opposite charge, so dextran sulfate does not only coacervate with chitosan, it also does it with the antigen. Furthermore, dextran sulfate induces colitis due to a reduction of the mucus layer. This reduction may allow the interaction of the released antigen in the lumen of the gut directly with the enterocytes and M-cells, potentiating the immune system.

According to another embodiment, the invention relates to another specific selection of the at least two anionic polymers, said other selection consisting in fucoidan and dextran sulfate.

In a further embodiment, the selection of at least two anionic components of the invention consists in dextran sulfate and TPP, or in dextran sulfate and carboxymethylcellulose sodium, or in dextran sulfate and poly(acrylic acid) based polymer, or in dextran sulfate and pectin, or in dextran sulfate and xanthan gum.

In a further preferred embodiment, the selection of at least two anionic components of the invention consists in dextran sulfate, alginate and TPP as anionic components, or in dextran sulfate, alginate and carboxymethylcellulose sodium, or in dextran sulfate, alginate and poly(acrylic acid) based polymer, or in dextran sulfate, alginate and pectin, or in dextran sulfate, alginate and xanthan gum or in dextran sulfate, fucoidan and TPP.

Immunogenic compositions and vaccines encompassed by the present invention can further include one or more pharmaceutically-acceptable carriers, which include any and all solvents, dispersion media, coatings, adjuvants, stabilizing agents, diluents, preservatives, antibacterial and antifungal agents, isotonic agents, adsorption delaying agents, antioxydants, colorants and the like. Diluents can include water, saline, dextrose, ethanol, glycerol, and the like. Isotonic agents can include sodium chloride, dextrose, mannitol, sorbitol, and lactose, among others known to those skilled in the art. Stabilizers include albumin, among others known to the skilled artisan. Preservatives include merthiolate, among others known to the skilled artisan.

In a preferred embodiment, the microparticles of the invention are devoid of any type of oils and fats including vegetable, animal or synthetic oils and fats.

The microparticles of the invention are also preferably devoid of any type of emulsifiers including synthetic emulsifying agens (cationic, anionic and non ionic, such as sorbitan esters (Spans^{®}), polyoxyethylene derivatives of sorbitan esters (Tweens^{®}), or glyceryl esters), natural emulsifying agents (vegetable derivatives, e.g., acacia, tragacanth, agar, pectin, carrageenan, lecithin... animal derivatives, e.g., gelatin, lanolin, cholesterol... semi-synthetic agents, e.g., methylcellulose, carboxymethylcellulose... synthetic agents, e.g., Carbopols^{®}), finely divided or finely dispersed solid particle emulsifiers (such as bentonite, veegum, hectorite, magnesium hydroxide, aluminum hydroxide and magnesium trisilicate...).

### Pharmaceutically active agents

An immunogenic agent according to the invention may be a virus, a bacteria, an immunostimulant or any antigen.

Specific agents that may be added to the oral vaccines described herein include, but are not limited to, *Aeromonas hydrophila, Aeromonas salmonicida*, *Pseudomonas fluorescens, Vibrio anguillarum*, *Vibrio salmonicida, Renibacterium salmoninarum*, infectious haemopoietic necrosis virus (IHNV), infectious salmon anemia virus (ISAV), pancreas disease virus (PDV) in particular, salmon alphavirus (SAV), salmon poxvirus, and viral haemorrhagic septicemia virus (VHSV).

Diseases of cultivated fish that may be prevented and/or treated using a composition for oral administration to an animal for intestinal delivery of the invention include, but are not limited to, Salmon Rickettsial Syndrome (SRS), infectious pancreatic necrosis (IPN), infectious hematopoietic necrosis (IHN), Vibriosis (Vibrio anguillarum), cold-water vibriosis (Vibrio salmonicida), Vibrio ordalii, winter ulcer (Vibrio viscosus), Vibrio wodanis, yersiniosis (Yersinia ruckeri) or Enteric Red Mouth, Bacterial Kidney Disease, Fumnculosis (Aeromonas salmonicida subsp. salmonicida), Saddleback, Gafkemia, Dollfustrema vaneyi, Cryptobia bullocki, Cryptobia salmositica, Listeria monocytogenes, Photobacterium damsela subsp. piscicida, and Microcotyl sebastis.

Therefore, compositions for oral administration to an animal for intestinal delivery of the invention are useful for immunization of fin-fish species against a variety of pathogens and diseases. Such pathogens and diseases include, but are not limited to, hemorrhagic septicemia virus (HSV); epizootic hematopoietic necrosis virus (EHNV); infectious hematopoietic necrosis virus (IHNV); Oncorhyncus masou virus disease ((OMV) also known as Yamame tumor virus (YTV)); Nerka virus Towada Lake, Akita & Amori prefecture (NeVTA); Coho salmon tumour virus (CSTV); Oncorhynchus kisutch virus (OKV); Coho salmon herpesvirus (CSHV); rainbow trout kidney virus (RKV); rainbow trout herpesvirus (RHV); infectious pancreatic necrosis virus (IPNV); fish pancreas disease virus (FPDV); spring viremia virus (SVV); yellowtail ascites virus (YAV) channel catfish virus disease (CCVD) (Herpesvirus ictaluri); grass carp hemorrhagic virus (GCHV); Nodaviridae causing viral encephalopathy and retinopathy (VER); viral nervous necrosis (VNN); infectious salmon anaemia virus (ISAV); Aeromonas salmonicida causing furunculosis; Vibrio salmonicida causing Vibrio salmonicida septicemia (VSS) or Hitra disease; Renibacterium salmoninarum; Yersinia ruckeri; Pasteurella sp.; Photobacterium damsela; Vibrio anguillarum; Vibrio ordalii; Moritellaviscosa, Edwardsiella ictaluri; Edwardsiella tarda; Streptococcus sp.; Lactococcus sp.; Flavobacterium sp.; Flavobacterium sp. Ichthyophthirius spp.; Lepeophtheirus sp. or Caligus sp. (sea lice); Bacterial kidney disease (BKD); Whirling Disease (Myxobolus cerebralis); Ceratomyosis (Ceratomyxa shasta); proliferative kidney disease (PKD); proliferative gill disease (hamburger gill); herpes virus salmonis (HVS); epizootic ulcerative syndrome (EUS); enteric septicaemia of catfish (ESC); Piscirickettsiosis (Piscirickettsia salmonis); Gyrodactylosis (Gyrodactylus salaries); Iridovirus causing red sea bream iridovirus (RSIV); grouper iridovirus (GSV-1 or GSV-2) or other ranaviruses such as sturgeon iridoviral disease; Cardiomyopathy syndrome (CMS), and heart and skeletal muscle inflammations.

Viruses encompassed by the present invention can be propagated in cells, cell lines and host cells. Said cells, cell lines or host cells can be for example, but not limited to, mammalian cells and non-mammalian cells, including insect, fish and plant cells. Cells, cell lines, and host cells in which viruses encompassed by the present invention can be propagated are readily known, and accessible to those of ordinary skill in the art.

Bacteria encompassed by the present invention can be cultured and propagated using various culture media known to those of ordinary skill in the art, including both broth (liquid) and agar (solid; semi-solid) cultivation media. Some bacteria can also be cultured and propagated in mammalian cells or non-mammalian cells.

The viruses and bacteria encompassed by the present invention can be attenuated or inactivated prior to use in an immunogenic composition or vaccine. Methods of attenuation and inactivation are well known to those skilled in the art. Methods for attenuation include, but are not limited to, serial passage in cell culture on a suitable cell line (viruses and some bacteria), serial passage in broth culture (bacteria), ultraviolet irradiation (viruses and bacteria), and chemical mutagenesis (viruses and bacteria). Methods for viral or bacterial inactivation include, but are not limited to, treatment with formalin, betapropriolactone (BPL) or binary ethyleneimine (BEI), or other methods known to those skilled in the art.

Adjuvants can be included in the vaccines described. The adjuvants which can be included in the vaccines described herein can be metabolizable, referring to adjuvants consisting of components that are capable of being metabolized by the target species such as vegetable oil based adjuvants. A metabolizable adjuvant can be a metabolizable oil. Metabolizable oils are fats and oils that typically occur in plants and animals, and usually consist largely of mixtures of triacylglycerols, also known as triglycerides or neutral fats. These nonpolar, water insoluble substances are fatty acid triesters of glycerol.

Triacylglycerols differ according to the identity and placement of their three fatty acid residues or side chains.

The adjuvant can also be non-metabolizable, referring to adjuvants consisting of components that cannot be metabolized by the body of the animal subject to which the emulsion is administered. Non-metabolizable oils suitable for use in compositions of the present invention include alkanes, alkenes, alkynes, and their corresponding acids and alcohols, the ethers and esters thereof, and mixtures thereof. Preferably, the individual compounds of the oil are light hydrocarbon compounds, i.e., such components have 6 to 30 carbon atoms. The oil can be synthetically prepared or purified from petroleum products. Preferred non-metabolizable oils for use in compositions described herein include mineral oil, paraffin oil, and cycloparaffins, for example. The term "mineral oil" refers to a non-metabolizable adjuvant oil that is a mixture of liquid hydrocarbons obtained from petrolatum via a distillation technique. The term is synonymous with "liquefied paraffin", "liquid petrolatum" and "white mineral oil." The term is also intended to include "light mineral oil," i.e., oil which is similarly obtained by distillation of petrolatum, but which has a slightly lower specific gravity than white mineral oil. Mineral oil can be obtained from various commercial sources, for example, J.T. Baker (Phillipsburg, PA), USB Corporation (Cleveland, OH). Light mineral oil is commercially available under the name DRAKEOL^{®}. Adjuvants include, but are not limited to, the Emulsigen adjuvant system (MVP Laboratories; Ralston, NE), the RIBI adjuvant system (Ribi Inc.; Hamilton, MT), alum, aluminum hydroxide gel, oil-in water emulsions, water-in-oil emulsions such as, e.g., Freund's complete and incomplete adjuvants, Block copolymer (CytRx; Atlanta, GA), SAF-M (Chiron; Emeryville, CA), AMPHIGEN^{®} adjuvant, saponin, Quil A, QS-21 (Cambridge Biotech Inc.; Cambridge, MA), GPI-0100 (Galenica Pharmaceuticals, Inc.; Birmingham, AL) or other saponin fractions, monophosphoryl lipid A, Avridine lipid-amine adjuvant, heat-labile enterotoxin from E. coli (recombinant or otherwise), cholera toxin, muramyl dipeptide, squalene/pluronic block copolymer/surfactant (SP-oil), sulpholipobeta-cyclodextrin (SL-CD), liposomes containing an immumodulator (e.g., CpG or poly I:C), muramyl dipeptide (MDP), iscomatrix (Quil A/phosphotidyl choline), CpG/DEAE-dextran/mineral oil (TXO), CpG, triterpenoids (e.g., Quil A or another purified or partially purified saponin preparation), sterols (e.g., cholesterol), immunomodulatory agents (e.g., dimethyl dioctadecyl ammonium bromide - DDA), polymers (e.g., polyacrylic acid such as CARBOPOL^{®}), and Th2 stimulants (e.g., glyco lipids such as Bay R1005^{®}), and combinations thereof, among many other adjuvants known to those skilled in the art.

Immunogenic compositions and vaccines encompassed by the present invention can include other additional antigens. Antigens can be in the form of an inactivated whole or partial preparation of the microorganism, or in the form of antigenic molecules obtained by genetic engineering techniques or chemical synthesis. Other antigens appropriate for use in accordance with the present invention include, but are not limited to, those derived from pathogenic viruses such as IPNV.

### Divalent ions

The oral bioadhesive delivery composition according to the invention may also comprise at least one divalent ion, such as magnesium, zinc and calcium. In one preferred embodiment of the present invention, the divalent ion is calcium.

Preferably, calcium is present in the compositions when at least one of the at least two anionic components is alginate. The divalent ions interact with the negative charges of the anionic polymer which enables the formation of a hydrogel by ionotropic gelation, therefore entrapping the antigen.

Preferably, calcium in the composition is in the form of calcium chloride.

It has been observed that increasing the calcium chloride concentration appears to enable a more rapid crosslinking at the level of the membrane of the particles.

In a another preferred embodiment, the calcium chloride concentration is comprised between about 0,1% and 15%, preferably between about 0,5% and about 10%. In a most preferred embodiment the calcium chloride concentration is comprised between about 1% and about 10%.

### Lyophilisation agent

The oral bioadhesive delivery composition according to the invention can optionally comprise at least one lyophilisation agent.

Said lyophilisation agent may be selected in the group comprising but not limited to saccharose, glucose, trehalose, glutamate, glycerol, mannitol, sorbitol, inulin, dextrin, dextran and gamma-polyglutamic acid.

Preferably, the lyophilisation agent according to the invention is a sugar. Said sugar enables to modify the density of the final composition but also enables to protect the antigen during the freeze drying step, if any. Said optional sugar is preferably sucrose, also known as saccharose. The optional sugar is preferably used in a concentration comprised between 1% and 30%, more preferably between 10% and 25% and even more preferably the concentration of the sugar solution is 20%.

### Immunostimulant

The oral bioadhesive delivery composition according to the invention can also optionally comprise an immunostimulant.

An example of a preferred immunostimulant includes, but is not limited to, beta-glucans. The main purpose of beta-glucans is to activate the immune system. Furthermore, it also has a role in the stability of the composition considering its low solubility in aqueous solutions.

Beta-glucans may be added to the composition. Initial concentration of the immunostimulant may be comprised between 0,01% and 1% w/v, preferably 0,05% and 0,5% w/v and even more preferably the concentration is comprised between 0.05 and 0.1% w/v. Even more preferably the concentration is 0,085% w/v.

### Method of treatment

Any reference in the present specification to a method of treatment is to be construed as a reference to a composition for use in said method of treatment.

The specification encompasses a method for vaccinating or boostering animals consisting in administering orally for the intestinal delivery to said animals an immunogenic composition comprising:
a) at least one mucoadhesive cationic polymer that is chitosan;
b) at least two anionic components, at least one of the two anionic components being dextran sulfate;
c) at least one immunogenic agent;
including all described embodiments of this immunogenic composition.

Protection against disease due to the method is considered to have been elicited when complete or partial immunity against the disease has been obtained. For example, when the method is applied in fish, immunity is considered as having been obtained in a population of treated fish when the level of protection for the population, evidenced by a decrease in the number of infected fish or in severity of disease, is higher in fish that have been treated in accordance with the invention than that of an unvaccinated control group. Preferably, vaccination in accordance with the method of the invention will result in a decrease of 20% in mortality due to the disease or in the number of individuals showing clinical signs of the disease compared to unvaccinated controls.

Immunogenic compositions and vaccines of the present invention include a therapeutically effective amount of one or more of the above-described microorganisms. Purified viruses and/or bacteria can be used directly in an immunogenic composition or vaccine, or can be further attenuated, or inactivated. Typically, an immunogenic composition or vaccine contains between about 1 × 10 ² and about 1 × 10 ¹² viral or bacterial particles, or between about 1 × 10 ³ and about 1 × 10 ¹¹ particles, or between about 1 × 10 ⁴ and about 1 × 10 ¹⁰ particles, or between about 1 × 10 ⁵ and about 1 × 10 ⁹ particles, or between about 1 × 10 ⁶ and about 1 × 10 ⁸ particles. The precise amount of a microorganism in an immunogenic composition or vaccine effective to provide a protective effect can be determined by a skilled artisan. In accordance with the methods of the present invention, a single dose can be administered to animals, or, alternatively, two or more inoculations can take place with intervals of from about two to about ten weeks. Boosting regimens can be required, and the dosage regimen can be adjusted to provide optimal immunization. Those skilled in the art can readily determine the optimal administration regimen.

The composition for oral administration to an animal for intestinal delivery of a pharmaceutically active agent according to the present invention is in a form of dry or semi-dry microparticles having a size range from 1 µm to 1000 µm, more preferably from 5 µm to 900 µm, more preferably from 10 µm to 800 µm, more preferably from 20 µm to 500 µm, more preferably from 90 µm and 250 µm. Mean size of the particles d(0,5) can advantageously be about 10 µm, about 20 µm, about 30 µm, about 40 µm, about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 190 µm, about 200 µm, about 220 µm, about 240 µm, about 260 µm, about 280 µm, about 300 µm, about 330 µm, about 360 µm, about 390 µm, about 440 µm, about 480 µm, about 500 µm.

Without wishing to be bound by theory, it is believed that the mean particle size of the microparticles plays a role in the reaching of the target in the animal and in the global efficacy of the treatment. It appears that the smaller size (order of the µm) are not necessary to obtain the better result in term of efficacy and stability of the composition. Indeed, relatively large particles (order of the dozens of µm to few hundreds of µm), that contains pharmaceutically active agents that are completely embedded in the material made of the at least two anionic components, the mixture being covered by the mucoadhesive cationic polymer, would benefit from a greater protection against the deleterious environment than particles of small size that would contain less pharmaceutically active agents, more rapidly in contact with the deleterious external agents or conditions like acids or enzymes. Furthermore, large particles that needs to be broken for having a size compatible to the oral administration could lose their integrity and could expose the pharmaceutically active agents to the deleterious external agents or conditions. An equilibrium has to be found for the size of the particles that fits with protection of the pharmaceutically active agents and their activity. A convenient particle size of integer particles could be comprised between 50µm to 500 µm.

The present invention also relates to a composition for oral administration to an animal for intestinal delivery of a pharmaceutically active agent comprising:
a) at least one mucoadhesive cationic polymer that is chitosan;
b) at least two anionic components, at least one of the two anionic components being dextran sulfate;
c) at least one immunogenic agent,
including all described embodiments of this composition,
for use in the vaccination or boostering of an animal against a pathogen.

In another preferred embodiment the animal is a fish.

According to a disclosure in the non therapeutic field that is not part of the present invention, the present specific relates also to a composition for oral administration to an animal for intestinal delivery of an agent comprising:
a) at least one mucoadhesive cationic polymer that is chitosan;
b) at least two anionic components, at least one of the two anionic components being selected in the group consisting in dextran sulfate, alginate and fucoidan;
c) at least one agent,
including all described embodiments of this composition,
for use in an animal.

Said agent can be selected in the group constituted by the dyes, growth factors, vitamins, hormones or any other agent susceptible to impact the overall health and organoleptic properties of an animal.

### Method for preparing a composition according to the invention

The present specification also relates to a general method for preparing a composition for oral administration to an animal for intestinal delivery of a pharmaceutically active agent that may be used to prepare a composition according to the invention.

Such general method to prepare the oral bioadhesive composition of the invention, that is in the form of microparticles, comprises the steps of
a. preparing an acidic aqueous solution, or first liquid mixture, comprising at least one bioadhesive cationic polymer, wherein the bioadhesive cationic polymer is preferably the mucoadhesive cationic polymer chitosan, and the acidic solution has a pH low enough to gelatinize the bioadhesive cationic polymer; preferably, pH is comprised between 5 and 6 ; more preferably, pH is about 5,5;
b. Preparing a second liquid mixture comprising at least two anionic components;
c. Mixing the pharmaceutically active agent and optionally other components in the second liquid mixture of step b);
d. Combining the first liquid mixture of bioadhesive cationic polymer of step a) to the second liquid mixture of anionic components obtained in step c) to form particles;
e. collecting the particles obtained from step d) and drying said particles;

Said anionic components are as defined previously.

At least one of the two anionic components is dextrane sulphate. Preferably the other anionic component is selected from alginate or fucoidan.

The combination of step d) of the first liquid mixture of bioadhesive cationic polymer of step a) to the second liquid mixture obtained in step c) in order to form particles can be made by dripping (or any equivalent means) the first liquid mixture containing the bioadhesive cationic polymer into the second liquid mixture containing at least two anionic components. Alternatively, the second liquid mixture containing at least two anionic components may be introduced into first liquid mixture of bioadhesive cationic polymer by any mean known to the skilled person in the art.

According to the method for oral bioadhesive composition of the invention, the other components according to step c) are immunostimulants, divalent ions, lyophilisation agent, adjuvants, buffers, one or more pharmaceutically-acceptable carriers, which include any and all solvents, dispersion media, coatings, stabilizing agents, diluents, preservatives, antibacterial and antifungal agents, isotonic agents, adsorption delaying agents, and the like. Diluents can include water, saline, dextrose, ethanol, glycerol, and the like. Isotonic agents can include sodium chloride, dextrose, mannitol, sorbitol, and lactose, among others known to those skilled in the art. Stabilizers include albumin, among others known to the skilled artisan. Preservatives include merthiolate, among others known to the skilled artisan.

Compositions according to the invention prepared according to this method of preparation are disclosed in the examples.

The step e) of collection of the particles is conducted by any means known to the skilled person in the art, as for example by centrifugation followed by elimination of the excess water through a draining sieve. The drying procedures are also known by the person skilled in the art. This procedure can consist in putting the particles in a drying oven (preferably at temperature not harmful to the pharmaceutically active agent or any other constitutive agent). Additionally or alternatively, freeze drying is used and in such a case the particles that advantageously pre-treated in a bath of lyophilisation excipient. In a proffered embodiment for the freeze drying procedure, the bath of lyophilisation excipient contains 20% of sucrose.

It has been observed that a satisfactory dispersion is quite difficult to obtain at an industrial scale at step d) and the resulting compositions can present problems of homogeneity and particles size due to the poor dispersion efficiency of traditional mixing devices. As a result, the average particle size d(0.5) is comprised between about 600 µm and 1000 µm.

During the manufacturing process it is also observed that the particles tend to re-aggregate along the drying process, forming large solid cakes. It is then necessary to introduce a grinding step in order to get particles which size is adaped to the tractus of the target species. For example, it is considered that a size comprised between 90 µm and 250 µm is particularly well fitted to the smolt salmons. As a consequence, the resulting grinded particles may present a non homogeneous coating with less mucoadhesive properties given that the original particles have a larger size and are chopped by the grinding process.

The method may further includes a step of mixing the second liquid mixture obtained in step c) with a divalent ion salt solution before step d) of combining the first liquid mixture of bioadhesive cationic polymer of step a) to the second liquid mixture of anionic components obtained in step c) to form particles. Said intermediate step enables the formation of a hydrogel.

One of the at least two anionic components may be alginate and the divalent ion salt may be a calcium salt. Therefore, in a preferred method, the hydrogel is formed by contacting the second liquid mixture of anionic components obtained in step c) with a calcium salt solution.

Said method is used to prepare a composition for oral administration to an animal for intestinal delivery of a pharmaceutically active agent, which is an immunogenic agent, whereby the composition comprises chitosan, at least two anionic components and where at least one of the at least two anionic components is dextrane sulfate. Even more preferably, the at least one other anionic component is selected in the group consisting in alginate and fucoidan.

Compositions according to the invention prepared according to this method of preparation are disclosed in the examples.

In a preferred method, the ratio of the at least two anionic components from the second liquid mixture in step c) to the solution of divalent ion salt from step d) is preferably 2:1.

In a preferred method, the divalent ion salt is calcium.

In another preferred method, the ratio from the hydrogel obtained in the second liquid mixture to the first liquid mixture from step a) is 1:1, 10:9, 8:2, 7:3, 6:4, 4:6, 3:7, 2:8 or 9:1.

It was observed that the hydrogels tend to be inhomogeneous. "Pre-particles" are observed that tend to sediment. In viscosity measurements, the measured values tend to decrease over time because of the sedimentation.

The inventors further demonstrated that the homogenization of the hydrogel is an important parameter for the efficiency of the final product since a good repartition of the pharmaceutical active agent is mandatory. The use of mechanical dispersion during or after the formation of the hydrogel especially enabled to homogenize the preparation.

Therefore, in a preferred method, the hydrogel is formed by contacting the second liquid mixture of step c) with a divalent ion solution under mechanic dispersion/agitation/homogenization.

In another preferred method, the hydrogel is placed under mechanical dispersion before being combined according to step d) to the first liquid mixture containing the cationic polymer solution of step a).

Said mechanical dispersion can be provided by commonly used dispersion tools, such as but not limited to, ultra turrax. Any equipment creating shearing and turbulence in order to favour diagregation, mixing and dispersion: tank equipped with a rotor-stator element, in-line static mixer, ultrasonic devices, etc.

### Feed

The present invention further relates to an animal feed comprising the oral bioadhesive composition for oral administration to an animal for intestinal delivery of a pharmaceutically active agent according to the invention; said composition can be sprayed onto said feed.

The present invention also relates to a method of preparing an animal feed consisting of blending the composition for oral administration to an animal for intestinal delivery of a pharmaceutically active agent according to the invention with a vegetable oil and top-coating feed pellets.

Also disclosed, but not claimed, is a feed regime wherein animals are fed with a composition for oral administration to an animal for intestinal delivery of a pharmaceutically active agent, for the oral vaccination of animals.

In a preferred embodiment, the vaccinated animal is a fish and, in a more preferred embodiment the fish are salmonids and the oral vaccination and/or booster, is to prevent the disease known as SRS.

### Exemples

### Example 1: preparation of a composition according to the present invention comprising chitosan, alginate and dextran sulfate

### Materials

- Distilled water (H2Od)
- Chitosan (85-90% deacetylated)
- Sodium Alginate
- Dextran Sulfate (MW: 500.000)
- β-glucans
- Glacial acetic acid
- Sucrose
- Sodium Hydroxide (NaOH 10N)
- Antigen (Bacterin)

### Methods

→ Chitosan solution 1% pH 5.5: dissolution of chitosan in distilled water and glacial acetic acid and pH adjusted with NaOH
→ Calcium Chloride 1% solution: dissolve 1.17 g of calcium chloride in 117 mL of distilled water.
→ Pre-gelification step: dissolve 1.17 g of Sodium Alginate and 1.17 g of Dextran Sulfate in 234 mL of H2Od. Then add 200 mg of 0-glucans and the antigen (bacterin 1×10⁸ - 1×10^{8.5} per dosis). Then add 117 mL of CaCl2 solution. The calcium ions will interact with the carboxylic groups of alginate polysaccharide forming a semi-solid hydrogel, entrapping the antigen (bacterin) inside the polysaccharide network.
→ Ionotropic gelation and Particle formation: To Dextran Sulfate solution adds under strong agitation 350 mL of 1% chitosan solution pH 5.5. The particles are formed instantly. The d(0.5) for the particles formed is about 700 µm and d(0.9) is about 1350 µm.
→ Sucrose step and Freeze-drying: centrifugation and discard of the supernatant. Put the particles on a 20% sucrose solution. After the incubation period, collect the particles and remove the excess of water. Distribute the particles homogeneously over the freeze-dryer tray. The lyophilization program will last around 24h, obtaining a dry mass on each tray. Finally, the dry mass is grinded and is converted to a fine powder.

After the grinding process, the mean size of the particles d(0.5) is about 150 µm.

### Assay 1: Measurement of the encapsulation efficacy of the particles

A small-scale trial to measure the encapsulation efficacy of the particles according to Example 1 and containing an antigen has been conducted.

The supernatant fluid obtained after collecting the particles, has been analysed by flow cytometry and quantifying the residual antigen. In a second experiment, the collected particles have been observed by fluorescence microscopy, differentiating the encapsulated antigen from the particles.

Following the Example 1 methodology, three particles with a final volume of 50 mL each were prepared. Each dose is comprised in 6 mg of dry particles. The particles were prepared based on the following antigen dose:
1. Particles 10⁸ bacteria/dose
2. Particles 10⁹ bacteria/dose
3. Particles Control without bacteria

Antigen *Escherichia coli*, stained using Life Technologies' SYTO^{®}BC commercial kit, has been used in accordance with the manufacturer's instructions.

Flow cytometry and fluorescence microscopy:
Once the particles were formed with the stained antigen dose, the particles were recovered and prepared serial dilutions with the remaining supernatant fluid were made. Next, these were analysed by flow cytometry using Becton Dickinson's FACSCalibur^{™} equipment. The collected particles were observed by fluorescence microscopy.

The residual antigen obtained was measured by flow cytometry for:
1. Particles 10⁸ bacteria/dose: Approximately 100% encapsulation efficiency, with no bacteria detected in the supernatant fluid.
2. Particles 10⁹ bacteria/dose: Antigen was detected in the supernatant fluid at a range of 10⁵-10⁶ free bacteria.
3. Particles Control without bacteria: No bacteria were measured.

Conclusions:
- The process allowed to define that the particles according to the invention can encapsulate 100% of the antigen, using a dose of 10⁸ bacteria.
- Increasing the bacteria per dose (10⁹) decreased the antigen encapsulation efficiency, detecting between 10⁵ and 10⁶ bacteria in the supernatant fluid.

### Example 2: preparation of a composition according to the present invention comprising chitosan, fucoidan and dextran sulfate

### Materials

- Distilled water (H2Od)
- Chitosan (85-90% deacetylated)
- Fucoidan (purity > 85%)
- Dextran Sulfate (MW: 500.000)
- β-glucans
- Glacial acetic acid
- Sucrose
- Sodium Hydroxide (NaOH 10N)
- Antigen (Bacterin)

### Methods

→ Chitosan solution 1% pH 5.5: dissolution of chitosan in distilled water and glacial acetic acid and pH adjusted with NaOH
→ dissolution of 1.75 g of Fucoidan and 1.75 g of Dextran Sulfate in 350 mL of H2Od and addition of 200 mg of 0-glucans and the antigen (bacterin 1×10⁸ -1×10^{8.5} per dosis), each dose being comprised in 6 mg of dry particles.
→ Ionotropic gelation and particle formation: addition of 350 mL of chitosan solution at 1% pH 5.5. The particles are formed instantly.
→ Sucrose step and Freeze-drying: centrifugation and discard of the supernatant. Put the particles on a 20% sucrose solution. After the incubation period, collect the particles and remove the excess of water. Distribute the particles homogeneously over the freeze-dryer tray. The lyophilization program will last around 24h, obtaining a dry mass on each tray. Finally, the dry mass is grinded to a fine powder.

### Assay 2: Efficacy trial of oral bioadhesive compositions according to the invention in fish

The bacteria *Piscirickettsia salmonis* causes Salmon Rickettsial Syndrome (SRS), which affects the main intensively-farmed salmon species, Coho salmon (*Oncorhynchus kisutch*), Atlantic salmon (Salmo salar) and Rainbow trout (*Oncorhynchus mykiss*). It was first described in the 1980s and is the main bacterial disease causing significant losses to national aquiculture.

In this study, the inventors evaluated the protective efficacy of oral bioadhesive delivery compositions according to the invention against the bacteria *Piscirickettsia salmonis.*

A total of 100 fish were used, divided into 4 groups of 50 fish each. The fish were of the *Salmo salar* species, in the pre-smolt stage, with an average weight of 30 grams.

During the course of the trial, the fish were fed at 1% of their live weight and kept at a temperature of 15+/-1°C, with an average oxygen saturation of 100%. 3 oral formulations were used in this trial:
- Dextran-Alginate composition as disclosed in example 1
- Fucoidan-Dextran composition as disclosed in example 2
- an oral commercial vaccine (SRS oral commercial vaccine, Virbac-Centrovet, Chile), as a positive control

A group of unvaccinated fish was included as a negative control.

The different vaccines were delivered orally, mixed with food, over a period of 10 days. For this purpose, the fish were placed in separate pools for each group.

Once vaccination is complete and after 300 accumulated thermal units, the fish were challenged by intraperitoneal injection of a *Piscirickettsia Salmonis* GB1 inoculum administered in a volume of 0.1 mL per fish.

Accumulated mortality and Relative Percent Survival in each group has been measured and the results of this experiment demonstrated the superiority in terms of efficacy against SRS infestation of the Dextran-Alginate composition and Fucoidan-Dextran compared to the oral commercially available vaccine (data not shown).

### Assay 3: Evaluation of the efficacy of an oral bioadhesive composition comprising Dextran-Alginate according to the invention against Streptococcus equi in equines

*Streptococcus equi* is a Gram-positive, beta-haemolytic, Lancefield group C bacterium. It causes the disease equine adenitis (distemper, equine distemper or Strangles), has high morbidity and causes problems for horse breeders.

This study was intended to measure and evaluate the protection provided by an oral bioadhesive formulation according to the invention (Dextran-Alginate composition as prepared according to Example 1) by means of a cohabitation challenge.

A total of 10 horses (male and female) with an average age of 1.5 years was used and examined by at least one veterinary physician using clinical assays to rule out any signs of disease or infectious process, such as fever, emaciation, diarrhoea, respiratory discharge and wounds, etc.

A Dextran-Alginate composition as prepared according to Example 1 was used in the trial. The indicated dose per animal is 10⁸ CFU of deactivated S. *equi* in 60 mg of the excipient. The composition was supplied to horses using MediGel^{®}Sucralose as the delivery vehicle. The study included 2 groups, 1 of which was used to evaluate the Dextran-Alginate composition of Example 1 and one group was the unvaccinated control. The vaccine was administered using a primary immunisation, followed by a booster 14 days after the first vaccination.

The natural challenge was carried out by cohabitation 14 days after providing the booster. This involved the immunised horses living together with a horse with the disease equine adenitis for a period of 14 days. Once this period was complete, the necessary sampling and treatments were carried out.

Once the trial was complete, the veterinarian in charge classified the challenge as successful and highly infectious. The disease was spread by the diseased or Trojan horses to the immunised horses as well as to the unvaccinated controls.

A score table was prepared based on progression of the disease, as described by Waller *et al.* 2007 (Data not shown). A score was assigned in relation to the clinical symptoms presented by each challenged horse: 0 represent no clinical sign of infection.

The Dextran-Alginate composition prepared according to Example 1 gave good results, conferring protection from the challenge, reducing infection and, in some cases, showing no clinical signs of the disease and obtaining a score of 0. On the other hand, all horses in the control group, were not immunised and have developed the disease at different levels of severity. (Data not shown)

### Assay 4: Evaluation of the efficacy of an oral bioadhesive composition comprising Dextran-Alginate according to the invention against Mycoplasma hyopneumoniae (M. hyo) in swines

This trial aimed to evaluate the efficacy of an oral bioadhesive delivery composition according to the invention (Dextran-Alginate as prepared according to Example 1) against *Mycoplasma hyopneumoniae* (*M. hyo*) given to disease-free pigs in their food in a mixed challenge model (homologous and heterologous). 21 pigs aged 3 weeks were used, provided by a breeding centre that is free from *Mycoplasma hyopneumoniae* and that does not vaccinate against *M. hyo* in the production line or in the breeding stock.

The challenge inoculum was prepared from a mixture of two sources containing *M. hyo* (pure and homogenised lung culture):
- *M. hyo* culture inoculum: a dose of 10 mL (10⁷ bacteria/mL) per pig was used, quantified by flow cytometry (Bacteria Counting Kit, Invitrogen USA). To prepare the culture inoculum, 3 vials of the 25095 (ATCC) strain of *M. hyo* stored in liquid nitrogen were used. These were placed in a filtered T25 cell culture flask (25 cm²) containing 30 mL of FRIIS medium. This culture was stirred at 90 rpm at a temperature of 37°C for 2-3 days until the characteristic colour change due to change in pH is observed in the culture medium (from red to yellow).
- Lung-macerate inoculum: each pig was inoculated with 10 mL of lung macerate, which contained at least 10⁷/mL genome copies/equivalent of *M. hyo*, quantified by qPCR. Preparing the lung macerate required the use of lung samples with lesions associated with symptoms of Enzootic Pneumonia, caused by *Mycoplasma hyopneumoniae.* These lungs were obtained at least 1 week in advance from authorised abattoirs and were selected *in situ.* The lung slices were macerated until homogenised in sterile 1x PBS in a biosafety cabinet. Once the macerate was prepared, a purity test was performed, using PCR to detect other respiratory pathogens such as the swine flu virus, PRRS, *Actinobacillus pleuropneumoniae*, *Streptococcus suis, Haemophilus parasuis, Actinobacillus suis, Bordetella Bonchiseptica, Pasteurella multocida, Mycoplasma hyorinis, Mycoplasma sinoviae and Mycoplasma suis.* Once the lung macerate was tested, it was quantified by qPCR in accordance with the IDB *M.* hyo-specific Cellular Quantification Instructions and Quantification Appendix.
3 groups were tested during the trial:
- Group 1: 7 pigs aged 30 days, free from *M. hyo* and specific pathogens were immunised through their food for 10 consecutive days with the Dextran-Alginate vaccine composition as prepared in Example 1 against *M. hyo.* Fourteen days after completing the first vaccination period, the same animals were given the Dextran-Alginate vaccine composition as prepared in Example 1 through their food for a further 10 consecutive days. During each vaccination period, each pig was expected to consume at least 250 mg of vaccine/pig (200 mg Dextran-Alginate + 50 mg dry Quillay). Nineteen days after completing the second oral vaccination period, the group of animals were challenged by applying a dose based on a mixture of pure *M*. *hyo* culture and lung macerate from a pig that was previously affected by *M. hyo.*
- Group 2: 7 pigs aged 30 days, free from *M. hyo* and specific pathogens, that were not be vaccinated against *M. hyo.* Those pigs were challenged 8 weeks after the start of the trial by applying a dose based on a mixture of pure *M. hyo* culture and lung macerate from a pig that was previously affected by *M. hyo.*
- Group 3: 7 pigs aged 30 days, free from *M. hyo* and specific pathogens, that were not be vaccinated against *M. hyo* and were not be challenged with the mixture of pure *M. hyo* culture + lung macerate.

The vaccination was given to animals aged 30 days starting on day 1 of the trial, in which each vaccination period consisted of administering the vaccine through food for 10 consecutive days. This vaccination was antigenically equivalent to 10 injectable vaccine doses (equivalent to 10⁸ DNA copies). Each vaccination was added to the communal troughs located in each run, following the specifications given in the vaccination appendix attached to this protocol.

During each vaccination period, correct ingestion of the vaccine was ensured, so it was recommended to provide the estimated number of kilograms of food for daily consumption based on the animals' age and weight.
- The challenge procedure was carried at 12 weeks of age. The vaccinated animals were challenged 3 weeks after consuming the last vaccine dose. The challenge was carried out with animals previously anaesthetised with 0.22 mL/kg of a mixture of 2% Xylazine and Zoletil 100. Each pig was then inoculated with 10 mL of pure culture, then immediately inoculated with 10 mL of lung macerate, using a sterile endotracheal tube placed in the trachea of each pig following anaesthesia in both cases.

Several parameters were measured during the trial in order to check the efficacy of the Dextran-Alginate oral bioadhesive composition according to the invention, including clinical symptoms (such as the presence of diarrhoea, cough, sneezing, nasal secretions, weakness, fever and other issues that could indicate behavioural changes), temperatures, final weights, Daily weight gain (DWG) Average daily gain weight (ADGW), detection of *M. hyo* in swabs, histopathological lesions or ELISA tests, Feed Conversion Rate After Challenge and percentage of lung lesions.

The results demonstrated the efficacy of the oral Dextran-Alginate composition which showed a decreased percentage of lung lesions as compared to the unvaccinated group and allowed a reduction of the lesions and losses caused by *Mycoplasma hyopneumoniae.*

## Claims

1. Composition for oral administration to an animal for intestinal delivery of an immunogenic agent, said composition being in the form of microparticles and comprising:
a) at least one mucoadhesive cationic polymer that is chitosan;
b) at least two anionic components, wherein at least one of the two anionic components being dextran sulfate;
c) at least one immunogenic agent;
for use in the oral vaccination or boostering of an animal against a pathogen.

2. The composition for oral administration to an animal for intestinal delivery of an immunogenic agent according to claim 1, wherein the at least one other anionic component is selected in the group consisting in alginate and fucoidan.

3. The composition for oral administration to an animal for intestinal delivery of an immunogenic agent according to claim 2, wherein said at least one other anionic components is alginate.

4. The composition for oral administration to an animal for intestinal delivery of an immunogenic agent according to claim 3, wherein the composition further includes at least one divalent ion.

5. The composition for oral administration to an animal for intestinal delivery of an immunogenic agent according to anyone of claims 1 to 4, for use according to claim 1, wherein the animal is a fish and the pathogen is causing the Salmon Rickettsial Syndrome (SRS).

6. Animal feed comprising the composition for oral administration to an animal for intestinal delivery of an immunogenic agent according to anyone of claims 1 to 4.

7. Method of preparing an animal feed consisting in blending the composition for oral administration to an animal for intestinal delivery of a pharmaceutically active agent according to anyone of claims 1 to 4 with an oil and top-coating feed pellets.

## Patentansprüche

1. Zusammensetzung zur oralen Verabreichung an ein Tier zur intestinalen Abgabe eines immunogenen Mittels, wobei die Zusammensetzung in Form von Mikropartikeln vorliegt und umfasst:
a) mindestens ein mucoadhäsives kationisches Polymer, das Chitosan ist;
b) mindestens zwei anionische Komponenten, wobei mindestens eine der beiden anionischen Komponenten Dextransulfat ist;
c) mindestens ein immunogenes Mittel;
zur Verwendung bei der oralen Impfung oder Boosterung eines Tieres gegen einen Krankheitserreger.

2. Zusammensetzung zur oralen Verabreichung an ein Tier zur intestinalen Abgabe eines immunogenen Mittels nach Anspruch 1, wobei die mindestens eine andere anionische Komponente aus der Gruppe bestehend aus Alginat und Fucoidan ausgewählt ist.

3. Zusammensetzung zur oralen Verabreichung an ein Tier zur intestinalen Abgabe eines immunogenen Mittels nach Anspruch 2, wobei die mindestens eine andere anionische Komponente Alginat ist.

4. Die Zusammensetzung zur oralen Verabreichung an ein Tier zur intestinalen Abgabe eines immunogenen Mittels nach Anspruch 3, wobei die Zusammensetzung weiterhin mindestens ein zweiwertiges Ion enthält.

5. Zusammensetzung zur oralen Verabreichung an ein Tier zur intestinalen Abgabe eines immunogenen Mittels nach einem der Ansprüche 1 bis 4, zur Verwendung nach Anspruch 1, wobei das Tier ein Fisch ist und der Erreger das Lachs-Rickettsien-Syndrom (SRS) verursacht.

6. Tierfutter, umfassend die Zusammensetzung zur oralen Verabreichung an ein Tier zur intestinalen Abgabe eines immunogenen Mittels nach einem der Ansprüche 1 bis 4.

7. Verfahren zur Herstellung eines Tierfutters, das darin besteht, die Zusammensetzung zur oralen Verabreichung an ein Tier zur intestinalen Abgabe eines pharmazeutischen Wirkstoffs nach einem der Ansprüche 1 bis 4 mit einem Öl zu mischen und Futterpellets zu überziehen.

## Revendications

1. Composition pour administration orale à un animal pour la délivrance intestinale d'un agent immunogène, ladite composition étant sous forme de microparticules et comprenant :
a) au moins un polymère cationique mucoadhésif qui est le chitosane ;
b) au moins deux composants anioniques, l'un au moins des deux composants anioniques étant le sulfate de dextran ;
c) au moins un agent immunogène ;
pour son utilisation pour la vaccination orale ou le renforcement d'un animal contre un agent pathogène.

2. Composition pour administration orale à un animal pour la délivrance intestinale d'un agent immunogène selon la revendication 1, dans laquelle au moins un autre composant anionique est choisi dans le groupe constitué par l'alginate et le fucoidan.

3. Composition destinée à être administrée par voie orale à un animal pour l'administration intestinale d'un agent immunogène selon la revendication 2, dans laquelle ledit au moins un autre composant anionique est l'alginate.

4. Composition destinée à être administrée par voie orale à un animal pour la délivrance intestinale d'un agent immunogène selon la revendication 3, dans laquelle la composition comprend en outre au moins un ion divalent.

5. Composition pour administration orale à un animal en vue de l'administration intestinale d'un agent immunogène selon l'une des revendications 1 à 4, pour l'utilisation selon la revendication 1, dans laquelle l'animal est un poisson et l'agent pathogène est à l'origine du syndrome de la rickettsiose du saumon (SRS).

6. Aliment pour animaux comprenant la composition destinée à être administrée par voie orale à un animal pour l'administration intestinale d'un agent immunogène selon l'une des revendications 1 à 4.

7. Méthode de préparation d'un aliment pour animaux consistant à mélanger la composition pour administration orale à un animal pour l'administration intestinale d'un agent pharmaceutiquement actif selon l'une des revendications 1 à 4 avec une huile et à enrober le mélange pour former des granulés d'aliments pour animaux.
